(19) Europäisches Patentamt
European Patent Office
Office européen des brevets

(11) Publication number: **0 173 085 B1**

(12) **EUROPEAN PATENT SPECIFICATION**

(45) Date of publication of patent specification: **08.06.94**

(51) Int. Cl.$^5$: **C08G 18/81**, C08G 18/67, C08G 18/10, C07C 271/06, A61K 6/10

(21) Application number: **85109410.2**

(22) Date of filing: **26.07.85**

The file contains technical information submitted after the application was filed and not included in this specification

# (54) Chain extended urethane dimethacrylate and dental impression formation.

(30) Priority: **31.07.84 US 636136**

(43) Date of publication of application: **05.03.86 Bulletin 86/10**

(45) Publication of the grant of the patent: **08.06.94 Bulletin 94/23**

(84) Designated Contracting States: **CH DE FR GB IT LI NL SE**

(56) References cited:
**FR-A- 2 391 705**
**US-A- 3 928 299**
**US-A- 4 233 425**
**US-A- 4 320 221**

(73) Proprietor: **DENTSPLY INTERNATIONAL, INC.**
**570 West College Avenue**
**P.O. Box 872**
**York Pennsylvania 17405(US)**

(72) Inventor: **Hare, Pamela H.**
**13 Albury Avenue**
**Georgetown Delaware 19947(US)**

(74) Representative: **Wächtershäuser, Günter, Dr.**
**Tal 29**
**D-80331 München (DE)**

EP 0 173 085 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### Background of the Invention

This invention relates to a new composition of matter comprising a urethane di(meth)acrylate which is especially useful as a compound for use in dental compositions that accurately conform to dental surfaces to be recorded, and to a method of producing the urethane diacrylate.

Methods of forming dental impressions are well known as are dental impression materials that are capable of accurately reproducing the surface contours and dimensions of oral tissues required in preparing dental prostheses. Since anatomic structures and preparations for prosthetic appliances are usually undercut, preferred impression materials are elastic or rubbery, ranging from gels, such as agar or algin preparations, to elastomers, such as rubbers, silicones, and polyethers. The nonaqueous elastomers are preferred because of their extreme dimensional accuracy and their relative stability under ambient conditions. In spite of all the improvements which have characterized current dental impression materials, they are still greatly limited by clinical factors when they are used in vivo.

It is known to prepare elastomeric impression materials by taking two separate pastes (one containing a catalyst and the other containing an accelerator), placing measured amounts of each on a pad of parchment or polyethylene-coated paper and immediately mixing them with a spatula into a substantially homogeneous mass. Such impression materials must be used immediately after mixing; and while curing to set is rapid, it must be timed to allow placement by fast and slow dental practitioner: and because the curing time is built in, special problems cannot be controlled with any degree of accuracy by the dental practitioner. All parts of the impression polymerize at substantially the same time. Also, the act of mixing tends to introduce air bubbles into the viscous pastes and these bubbles are difficult to eliminate, tending to cause surface imperfections in the finished impression or distortion of the impression. Mixing is inconvenient and a source of inconsistency.

In the usual practice, a dental practitioner places the mixed paste in juxtaposition to the dental tissues, using either a supporting tray to contain the paste or a combination of a placement syringe and a supporting tray. The dental practitioner or dentist and the patient then wait, sometimes for ten minutes, for the polymerization reaction to progress to completion and the material to become sufficiently elastic so that the impression may be removed from the tissue without distortion of the remembered shape or form. The rate of faulty impressions is quite high due to the patient's natural tendency to move during this time, and a gagging reflex is common. The dental practitioner loses valuable time while he is thus inactivated, plus time needed for the often required retakes.

Materials commonly used for taking impressions are polysiloxanes such as described in United States Patent Number 3,950,300, polyethers such as described in United States Patent Number 3,453,242, and other elastomeric materials having properties more fully described in American Dental Association Specification 19.

US-A-3928299 relates to light-curable polymers comprising a polymeric backbone, which may be a polyurethane and which are crosslinkable by vinyl polymerization. These products are described as being useful for production of high quality copying layers for printing purposes.

US-A-4233425 describes radiation curable polymers having ethylenically unsaturated urethane groups. These products are employed as flooring material, i.e. coating compositions for substrates such as floor materials.

US-A-4320221 relates to adhesives which have to be used under unaerobic conditions.

FR-A-2391705 describes dental impression materials comprising an isocyanate dipolymer having both terminal sites end-capped with hydroxyalkyl(meth)acrylate.

According to the present invention, a new composition of matter is provided comprising a urethane di(meth)acrylate obtainable by reacting a saturated dihydroxy compound with a diisocyanate and forming a first reaction product having about 2 equivalents of reactive isocyanate, reacting said first reaction product with about 1 equivalent of a hydroxyalkyl(meth)acrylate preferentially reacting with said first reaction product to form a second reaction product, reacting said second reaction product with a glycol to form a third reaction product and reacting said third reaction product with an isocyanato alkylene (meth)acrylate.

The new urethane di(meth)acrylate provides an excellent dental composition component that is nontoxic in use in the oral cavity and will assume a permanent elastomeric memory when cured. When the new urethane di(meth)acrylate is provided with an initiator activated by actinic light within the visible light range of 360 to

600 nanometers it can be substantially stable against assuming a permanent remembered form when stored actinic light free, and then on exposure to light filtered to limited wave lengths within the visible light

range for one (1) minute cure to a depth of 2,54 cm (one (1) inch).

The urethane di(meth)acrylate is made according to the following method. First, a saturated dihydroxy compound is reacted with a diisocyanate to form a first reaction product having about 2 equivalents of reactive isocyanate. This first reaction product is then reacted with about one equivalent of a hydroxyalkyl-(meth)acrylate preferentially reacting with the first reaction product, to form a second reaction product. The second reaction product is then reacted with a glycol to form a third reaction product. This third reaction product is then reacted with an isocyanato alkylene(meth)acrylate.

The present invention in a preferred embodiment of one of its aspects is a new composition of matter comprising a new compound having the following general formula

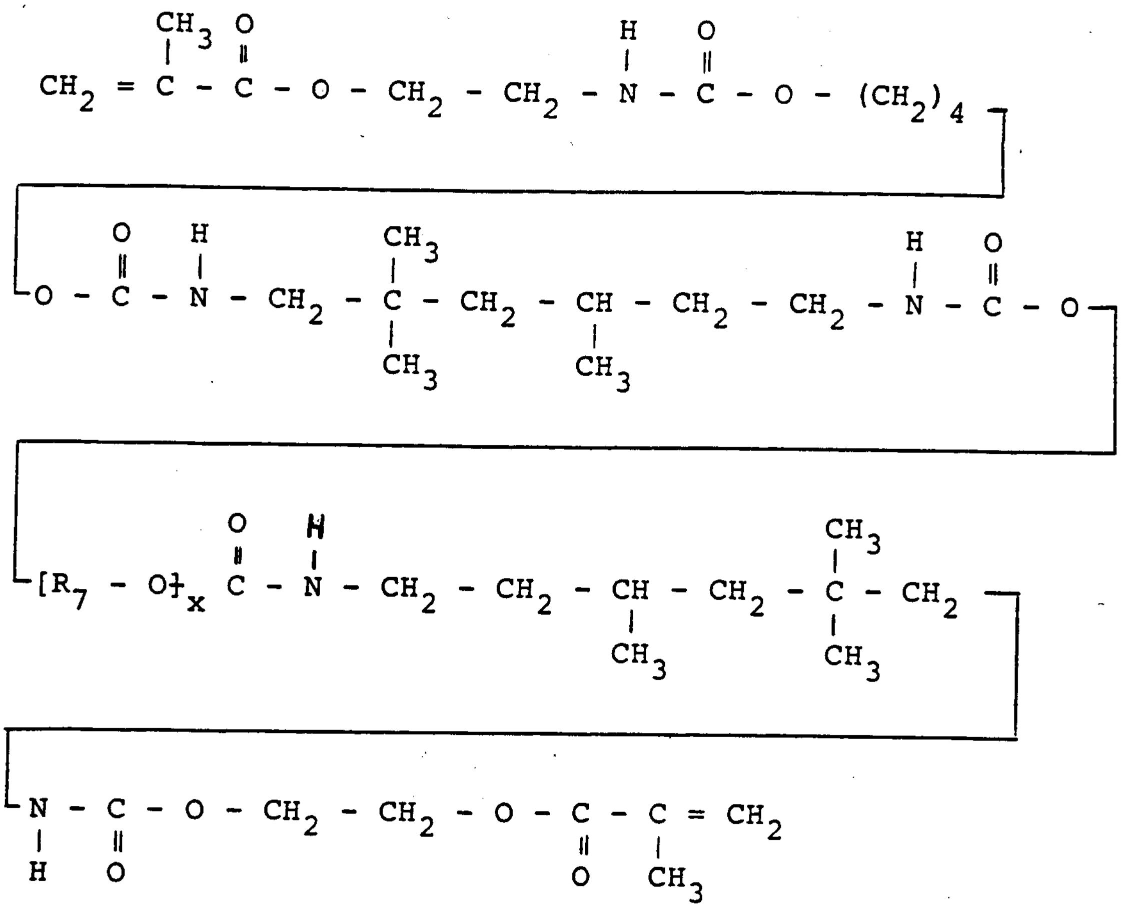

$R_7$ = Alkylene;
x = 10 to 100.

The new compound of the present invention is prepared by forming a first reaction product by reacting a saturated dihydroxy compound with a diisocyanate, then reacting the first reaction product with a hydroxyalkyl(meth)acrylate in an amount of approximately 1 equivalent, then this second reaction product is chain extended with a glycol and this third reaction product is then end capped at its chain extended end with an isocyanato alkylene(meth)acrylate.

The urethane di(meth)acrylates of this invention, can be made up of compounds of different molecular structure having the recited characteristics or of a quantity of a single molecular structure. The use of the term equivalents as used in the context of this patent application refers to the reactive potential under the conditions of the recited reaction.

The reacting of the second reaction product with the glycol is understood as a chain extending reaction at least predominantly capping the pendent reactive isocyanate group of the second reaction product. Preferably, the dihydroxy compound used to form the first reaction product is a dihydroxy oxyalkylene and the third reaction product preferably is reacted with an isocyanato alkylene acrylate.

The saturated dihydroxy compounds which are especially useful in preparing the first reaction product may comprise any of a wide variety of materials, especially the polyethers, polyesters and polycarbonates. One or more materials having two hydroxylic functionalities, which material is not ethylenically unsaturated

may be so employed. Preferred materials include aliphatic diols having from 8 to 20 carbon atoms between the hydroxylic functions such as dodecane diol or decane diol. Certain prepolymeric materials, such as the polyalkylene ether glycols are even more preferred. Accordingly, materials such as polymethylene ether glycol, polyethylene ether glycol, polypropylene ether glycol and polybutylene ether glycol may be so employed. It will be understood by those skilled in the art that a wide variety of such ether glycols may be useful in the practice of the present invention. Preferred materials for use in accordance with the present invention are polybutylene ether glycol (also called polytetramethylene ether glycol) and polypropylene ether glycol. As will be readily appreciated, the foregoing polyalkylene ether glycols are generally available as mixtures of species having differing molecular weights.

It will be appreciated that a wide variety of diisocyanates may be employed in preparing the first reaction product in accordance with the present invention. Exemplary species include hexamethylene diisocyanate, tetramethylhexamethylene diisocyanate, isophorone diisocyanate, the trimer of isophorone diisocyanate and the trimer of 1,6 hexamethylen diisocyanate. It has generally been found most preferred to employ diisocyanate species which are either aliphatic or cycloaliphatic in nature. While such non-aromatic diisocyanates are preferred, aromatic materials such as toluene diisocyanate and methylene bisphenyl-4-diisocyanate may also be used. The isocyanate functions of the foregoing materials may be viewed as being separated by a number of carbon atoms. Such number of carbon atoms is preferably from 6 to 20. A preferred diisocyanate for use in accordance with the present invention is trimethylhexamethylene diisocyanate.

The saturated dihydroxy compound is reacted with the diisocyanate in such a fashion as to always have isocyanate functions in stoichiometric excess over hydroxy functions. It is preferred that this excess be from 50% to 150% by mole with 75% to 125% being preferred. It is still more preferred that about 100% by mole of diisocyanate be included in excess over the molar amount of hydroxylic functions.

The hydroxyalkyl(meth)acrylates employed in the present invention to form the second reaction product may be chosen from a variety of compounds. Preferred hydroxyalkylation products of acrylic and/or methacrylic acid are acrylic acid hydroxyethyl ester, acrylic acid hydroxypropyl ester, methacrylic acid hydroxyethyl ester and methacrylic acid hydroxypropyl ester. The most preferred is methacrylic acid hydroxyethyl ester.

The second reaction product, which is basically a monoacrylated urethane, is chain extended with a glycol such as ethylene, propylene, diethylene or butylene glycol. The preferred chain extender is 1,4 butylene glycol.

The isocyanatoalkylene(meth)acrylate may be chosen from a variety of compounds. Preferred compounds are isocyanatoalkylene methacrylates and the most preferred is isocyanatoethylmethacrylate.

In accordance with this invention, the urethane di(meth)acrylates formed hereby are preferably included in compositions that are dental impression materials.

The dental impression material composition of the present invention is preferably substantially stable against assuming a permanent remembered form when stored actinic light free. The composition is preferably stable when stored as a single one-component material for a long period of time actinic light free, preferably being stable for at least one month, more preferably three months, and most preferably for six months or more. By one-component, it is meant that the dental impression material can be stored in the exact form that it will be used by the dentist so that he preferably does not need to do anything other than mold the composition to the surface (surfaces) that is to have its impression made.

The photoinitiating system may be one of many known in the art to promote polymerization of the unsaturated acryl groups of the urethane di(meth)acrylate used, when activated by actinic light of the appropriate wavelengths, strength and length of exposure time. Such systems include, but are not limited to camphoroquinone and other alpha-beta diketones, alone or with reducing agents, such as secondary and tertiary amines, compounds known to catalyze photopolymerization of acrylates upon irradiation by visible light. Materials such as benzoin and benzoin methyl ether which are known to be photopolymerization catalysts utilizing light in the UV portion of the electromagnetic spectrum are operable to cure the presently preferred polymers, but UV light is considered generally undesirable in most instances.

In the aspect of the present invention involving the actinic light activated photopolymerizing composition, the composition is for health and safety reasons preferably one that can be expeditiously cured using light filtered to limit the wave lengths to the visible light range of approximately 360-600 nanometer. More preferably the curing is, carried out with the greater portion of the light being within the 400-500 nanometer range.

The amount of photopolymerization sensitizers and catalyst and their type are selected with due consideration to the intensity of the light source and the activating wavelength(s) and their own capacity to initiate polymerization. Sensitizers, for example, camphoroquinone, may be typically used in concentrations

between 0.05 and 5% by weight of the polymerizable resin present, most preferably between 0.1 and 1.0%. Catalysts for the photopolymerization sensitizer, for example, tertiary amines, including, for example, methyldiethanolamine, diethanolamine, or triethanolamine may be used. These are preferably used in amounts of between 0.1 and 10% of the concentration of the total dental impression material composition, but most preferably between 0.1 and 5%.

Because the urethane di(meth)acrylate to be used in the dental impression material composition of the present invention is also an independent aspect of the present invention it is pointed out that the urethane di(meth)acrylate can be applied to the preparation of self curing dental impression using the more conventional types of curing systems employed in dental impression materials, such as, a catalyst paste containing as the initiator, benzoyl peroxide, with fillers such as quartz, talc and silica, a diluent such as polypropylene glycol with a molecular weight, for example, of 4,000 and a stabilizer such as BHT (butylated hydroxy toluene). The base paste would correspondingly contain an accelerator such as dihydroxyethyparatoluidine, a diluent such as polydimethylsiloxane and fillers. By "self curing" it is meant a dental impression material that in the form it is used in will cure at a predetermined rate due to built-in curing activaters that need no external initiation to the impression material, such as actinic light initiation.

The impression material can be a viscous liquid, or it can be modified with fillers to result in more viscous pastes or even putties. Such fillers should have suitable optical characteristics so as not to interfere with the transmission of actinic light through the material in order to initiate the photoinitiator system when photoinitiated. The filler particles should have size and surface area appropriate to effect the desired viscosity change.

Reinforcing fillers may also be used in the composition of the present invention. Preferred reinforcing fillers have a surface area of at least 50 square meters per gram and are exemplified by pyrogenically-produced silicon dioxide, silicic acid hydrogels dehydrated so as to maintain their structure, silicon dioxide Aerogels, and precipitated silicon dioxide. Other fillers may be used which are considered to be non-reinforcing fillers. Such fillers are generally those having a surface area less than 50 meters per square gram, and include calcium carbonate, fused quartz powder, powdered calcium silico aluminate, titanium dioxide, zirconium silicate and aluminum silicate. These materials may be ground or formed by a variety of means to provide particulate powdered filler of sizes between 0.001 and 100 $\mu$m, depending on the application. Particles of individual average sizes of 0.01 and 40 $\mu$m are especially preferred.

All of these fillers, but especially the reinforcing fillers, can have organosilyl groups on their surface if they have been pretreated, for example, with dimethylhalogen silanes, or if they have been manufactured, for example, by reaction of aqueous silica sol with organo halogensilanes, or have been rendered hydrophobic in some other way. Mixtures of different fillers can be used. Non-reinforcing fillers may be used at concentrations of at least 20% by weight relative to the prepolymers present, whereas reinforcing fillers may be appropriately used in the compound at from 1% to 80% by weight, relative to the total weight of all prepolymers present. In the case of actinic light cured compositions, an important consideration is that the amount and the type of filler is so selected that actinic irradiation may pass through the polymerizable mass in order that polymerization can occur upon irradiation to the depth of the impression but the filler need not match the refractive index of the resins exactly.

Organic resins, for example PVC powder or methacrylate polymer powder or polyethylene may be used as suitable extenders and plasticizers. The compositions of the invention may be stabilized by the addition of hydroquinone, catechol, and other similar well-known polymerization inhibitors for the polymerization of (meth) acrylate compounds. Other optional ingredients include pigments and flavoring substances. Still other plasticizers may include, for example, siloxanes, phthalates, azelates and glycerides. Such plasticizers are generally added to alter the hydrophobicity, the softness or hardness of the composition, its viscosity or tackiness.

The dental impression material or composition in the usual situation is preferably non-adhering to tooth enamel, amalgam, composite tooth fillings, metal bridgework, and other substances commonly found in a variety of different patients so that the composition can have relatively universal use. The composition should have the non-adhering ready release characteristic when, or that is after, it is changed from its flowable to its remembered form on being cured. The composition should also not harm soft tissue structure in the mouth of the patient, be substantially non-toxic in use, and not induce allergic reactions of substance in the patent population as a whole. The composition should be easily removed from the soft tissue also and accurately record the soft tissue shape in permanent elastomeric remembered form of impression.

The present invention in a preferred embodiment of one of its aspects is the utilization of the new composition of the present invention in a method of forming a dental impression in the oral cavity. First, the surfaces to be taken are cleaned and cleared of anything on them including mouth fluids. Then a

composition that is flowable, at least substantially free of memory and capable of assuming a permanent elastomeric memory in response to contact by actinic light is engaged with the surfaces that are to have their dental impression made. This includes in a preferred embodiment, forcing a tray of the composition toward the surface until some of the composition flows to assure a good engagement of the composition with the surfaces to be recorded. The tray is preferably maintained in contact with the composition to hold it securely in place, and actinic light is passed through at least an integral part of the tray activating the photopolymerizing of the composition to a degree that the composition assumes a permanent elastic remembered form.

A preferred tray passes actinic light through all of its mass to the composition. For this purpose, the tray may be a clear plastic.

In its preferred form, the method includes aspects of the materials that can perform the needed actions for preferred performance of the preferred methods of the present invention. The preferred method does not require pre-mixing of the composition before its use. The compositions are preferably flowable, deformable and substantially tree of any shape memory prior to activation by actinic light so that the composition can be formed to the dental, including the adjacent soft tissue, surfaces of the oral cavity. The preferred composition assumes a permanent elastomeric remembered form, in response to actinic light exposure. By permanent elastomeric remembered form, it is meant that the dental impression material can be stripped from the teeth by stretching and deforming in response to pressure applied by, in the usual usage, the human hand to pull the material off the teeth while retaining the remembered shape of the teeth in detail to which the material returns upon release of the pressure.

The one-component composition of the present invention can be packaged in various ways, including being preloaded into a syringe from which the dentist can express the material directly onto the soft or hard tissues to be reproduced. The composition can also be preloaded into a dental impression tray which can be placed by the dentist directly into the mouth of the patient, or can be preloaded into a collapsible tube from which the dentist can squeeze the material into a dental impression tray that passes actinic light prior to placement in the patient's mouth. An important point is for the container or its overwrap to be metal or otherwise opaque to actinic light, or be packaged in such a manner as to protect the composition of the invention from actinic light prior to use by the dentist.

In a preferred embodiment of the present invention the dentist places the special tray filled with the composition of the present invention in the mouth of the patient in such a way that the impression material fully contacts the entire area of the oral tissues of which an impression is being made. An optional step may be taken by the dentist prior to placing the filled tray in the patient's mouth in order to avoid entrapping air bubbles at the tissue surface or in constricted areas, the dentist would coat the surface of the tissues, especially constricted areas, such as between teeth, with a more fluid impression material of the present invention preferably by extrusion from a syringe, and then place the filled tray as described above.

After placement of the special tray, the impression material is polymerized by visible light within a minute, more preferably within 30 seconds and most preferably within 20 seconds or less, by a source of actinic light, such as a PRISMA-LITE™ polymerization unit of The L.D. Caulk Company, which produces visible light with a band of wavelengths between 400 and 500 nanometers and an energy output of approximately 400 milliwatts per square centimeter from the tip of the unit's light guide. The polymerization time can vary depending on the intensity and wavelength of the light used, the quantity of material to be polymerized, and the tray used. For example, the tray could be a special tray of the construction described below.

The time required for the dentist and the patient to wait for polymerization or setting of the shape to take place may be reduced from 8 - 10 minutes to one minute or less, and the total time required for placement and curing of the one-component impression material of the present invention may be reduced to 2-3 minutes, as compared to approximately 15 minutes in conventional techniques, which require mixing of two-component impression materials.

The impression tray to be used with the composition of the present invention must be capable of transmitting light to all areas of the impression material that are to be activated directly by the actinic light. One simple construction would be simply a standard transparent plastic tray, whereby polymerizing light can be directed through all portions of the base of the tray onto the material inside the tray.

A newly developed tray is the subject of a separate patent application, filed on the same day as this patent application, and assigned to the same assignee as the present patent application. This tray has a light guide means, such as a short solid light pipe rod at the anterior portion of a transparent tray which transmits light from the light source to the tray. The light is then transmitted to the impression material by the body of the tray itself. The light may be reflected or deflected directly into the material by a reflective tray surface. Such reflective surfaces are provided by metallized mirror-like coatings on the outer tray

surface, or by geometric shaped facets, grooves or ridges which reflect or deflect light at roughly 90° from the general surface of the tray. The facets, grooves or ridges occur either on the outer or inner tray surfaces.

A special impression tray could be prefilled with impression material and be wrapped entirely with a metal foil-plastic laminate material to be opened at an area allowing for the taking of the impression only at the time of use, which would prevent the impression material from being exposed to light before use. The metal foil could serve the dual function of preventing unintentional light exposure and subsequently providing a reflecting surface for the light supplied to the tray to bring about polymerization.

The preferred materials of the present invention have special applications in dentistry in addition to their most preferred application in preparing dental impressions. By dental impressions it is meant, reverse images of dental features in the mouth to serve as molds from which dental prosthesis can be prepared or models for preparing dental prosthesis can be prepared. The preferred materials of the present invention also have application in methods of directly preparing dental prosthesis by which term it is meant to include parts of dental prosthesis. This provides the format for an entirely new method of preparing dental prosthesis. A particularly preferred aspect of the present invention is the preparing of dental prosthesis by relining of dentures that are either damaged or no longer fit properly and/or comfortably.

The dentist would take a removable denture that is no longer fitting comfortably in a patient's mouth and apply to all of the areas of the denture that contact the patient's soft tissue, a thin coating of one of the compositions of the present invention. The dentist would then insert the denture into the patient's mouth and engage the composition while it is flowable and at least substantially free of memory with the surfaces in the oral cavity that are to be reproduced as the new closely fitting negative dental prosthetic part of the surface. The denture is pushed firmly into place, forcing the composition against the patient's dental surface until some of the composition flows into good conformity with the surface to form the composition into an accurate negative impression of the oral surface. The denture is then removed from the patient's mouth and inserted in to a TRIAD™ light curing unit (a product of Dentsply International Inc.) and actinic light is impinged on the negative impression formed composition by operation of the unit. This photopolymerizes the composition to a degree that the composition assumes a permanent elastomeric remembered form of the negative of the oral surface. It will be understood that the flowable composition is carried on the surface of the removable denture that is to be juxtaposed against the soft tissue in the oral cavity when said composition is forced toward the soft tissue surface. The actinic light, except for ambient light, is preferably substantially limited to the visible light spectrum of 360 to 600 nanometers. If on reinsertion everything is not as desired, adjustment can be easily made by stripping off the reline prosthesis or cutting out a portion of it and repeating the forming process directly to the soft tissue as described.

The composition of the present invention is preferably substantially stable against assuming a permanent remembered form when stored actinic light free. The composition is preferably non-toxic in use in the oral cavity, stable in storage for at least one (1) month as a one-component composition when actinic light free, and assumes a permanent elastomeric memory when exposed to light filtered to limited wave lengths within the visible light range for one (1) minute to a depth of one (1) inch (2,54 cm).

The invention is further illustrated by the following examples:

## EXAMPLE 1

A preferred isocyanatoethyl methacrylate urethane methacrylate oligomer compound was prepared according to the following formulation:

| | |
|---|---|
| Polypropylene glycol MW-2,000 Voranol 2120 (Dow Chemical) | 690 g |
| Trimethyl hexamethylene diisocyanate (Thorson) | 145 g |
| Dibutyl tin dilaurate | 0.4170 g |
| Hydroxyethylmethacrylate (HEMA) (Esschem) | 50.0 g |
| 1,4 Butane diol (BASF) | 31.0 g |
| Isocyanatoethyl methacrylate (Dow Chemical) | 53.4 g |

The procedure was as follows:

One mole of polypropylene glycol (2 equivalents of hydroxy) are reacted with two moles of trimethyl hexamethylene diisocyanate (4 equivalents of isocyanate) employing the dibutyl tin dilaurate.

The polypropylene glycol was dewatered with molecular sieve (4A) for two days. Then it was charged into a 2 liter reactor. Stirring and dry air flow through the reactor was begun. The dibutyl tin dilaurate was

added to the glycol dropwise and allowed to stir in. Then the diisocyanate was added to the glycol-catalyst mixture dropwise using a separatory funnel. The addition was done at room temperature and the drop rate was controlled to keep the temperature below 50 °C. After about three hours, all the diisocyanate had been added. The mixture was allowed to stir overnight with a heating mantle up around the reactor (no heat turned on). The next day 45 grams HEMA was added dropwise, again controlling the drop rate to keep the pot temperature below 50 °C. After all the HEMA was added, the 1,4 Butane diol was added dropwise to the reactor contents. This mixture was allowed to stir overnight. The next day, isocyanatoethyl methacrylate was added dropwise through the separatory funnel and stirred in. A slight excess of HEMA (5 grams) was finally added to the pot about three hours after the final addition of isocyanatoethyl methacrylate to be sure all the free isocyanate was reacted. The pot contents were allowed to stir for 24 hours and then unloaded.

EXAMPLE 2

A dental impression forming composition was compounded by hand mixing the following formulation at ambient conditions.

| | |
|---|---|
| Resin of EXAMPLE 1 | 100 parts by wt. |
| Camphoroquinone | 0.15 " " " |
| Methyl diethanol amine (MDEA) | 0.5 " " " |

The dental impression forming composition was then tested for its relevant characteristics with the following results:

The composition was irradiated with a 500 watt General Electric Photo-EBV photoflood lamp containing light from the visible light spectrum for 5 minutes with the lamp approximately 2 inches (5,08 cm) from the dental impression forming composition specimen. The material cured to an elastic solid. The following testing results were obtained using ADA Spec 19 (1984) when the cured dental impressions composition cured by irradiation as described was tested:

| Compression Set (%) | Strain (%) | Dimensional Change (%) |
|---|---|---|
| 0.65 | 3.75 | 24 hrs ± 0.23 expansion<br>1 wk ± 0.27 expansion |

EXAMPLE 3

A dental impression forming composition was compounded by hand mixing the following two formulations separately at ambient conditions.

The catalyst paste was prepared according to the following formulation.

| | |
|---|---|
| 1. resin | 52.05 parts by wt. |
| 2. benzoyl peroxide | 1.04 " " " |
| 3. filler | 27.33 " " " |
| 4. polypropylene glycol (MW 4,000) | 19.52 " " " |
| 5. BHT (butylated hydroxy toluene) | 0.06 " " " |

A base paste was prepared according to the following formulation:

| | | |
|---|---|---|
| 1. | resin | 51.80 parts by wt. |
| 2. | dihydroxy ethyl p-toluidine | 0.93 " " " |
| 3. | filler | 27.20 " " " |
| 4. | polypropylene glycol (MW 4,000) | 19.43 " " " |

The two pastes were mixed at an equal weight ratio by spatulating on a parchment pad for approximately 45 seconds. The material cured to an elastic solid with a shore A hardness of about 55 (ASTM 19, 1984 testing method) in 6 minutes at ambient temperature.

Below are the reults of testing this material in accordance with ADA Spec 19 for non-aqueous elastomeric impression materials:

| Dimensional Change % | | Mix (mm) Consistency | Compression Set (%) | Strain (%) | Flow (%) |
|---|---|---|---|---|---|
| 24 hrs.<br>1 wk. | 0.17<br>0.18 | 49 | 0.5 - 0.6 | 2.5 - 3.5 | 0.10 |

The material was determined to be compatible with gypsum and detailed reproduction was fine when tested in accordance with ADA Specification 19 for non-aqueous elastomeric materials.

## Claims

**1.** A new composition of matter comprising a urethane di(meth)acrylate obtainable by reacting a saturated dihydroxy compound with a diisocyanate and forming a first reaction product having about 2 equivalents of reactive isocyanate, reacting said first reaction product with about 1 equivalent of a hydroxyalkyl(meth)acrylate preferentially reacting with said first reaction product to form a second reaction product, reacting said second reaction product with a glycol to form a third reaction product and reacting said third reaction product with an isocyanato alkylene (meth)acrylate.

2. A new composition of matter according to Claim 1 comprising a compound of the general formula

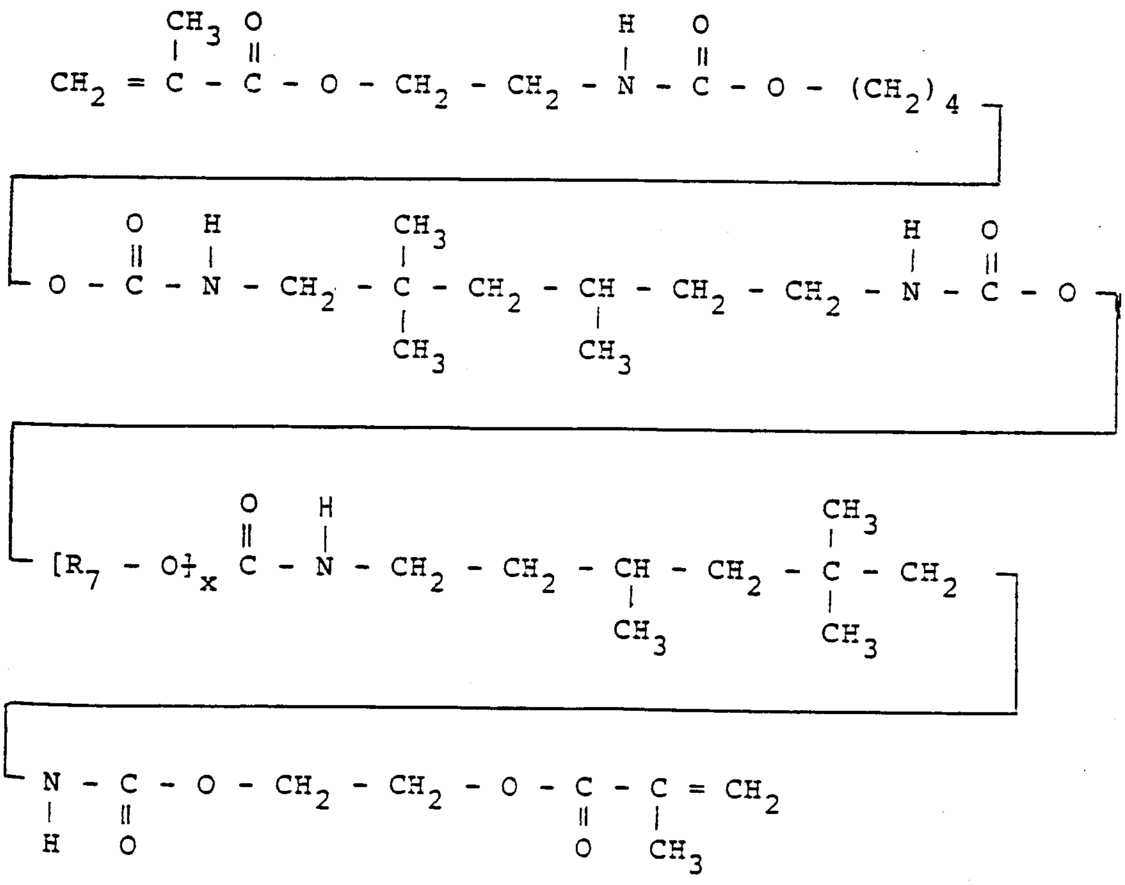

$R_7$ = Alkylene;
x = 10 to 100.

3. A composition of any of claims 1 or 2, which is formulated as a photo-curable dental impression material.

4. The new composition of matter of any one of claims 1 through 3 comprising an initiator activated by actinic light within the visible light range of 360 to 600 nanometers.

5. A method of producing a urethane di(meth)acrylate suitable for producing a composition of claim 1, comprising reacting a saturated dihydroxy compound with a diisocyanate and forming a first reaction product having about 2 equivalents of reactive isocyanate, reacting said first reaction product with about 1 equivalent of a hydroxyalkyl(meth)acrylate preferentially reacting with said first reaction product to form a second reaction product, reacting said second reaction product with a glycol to form a third reaction product and reacting said third reaction product with an isocyanato alkylene (meth)acrylate.

6. The method of Claim 5, wherein said isocyanato alkylene (meth)acrylate is isocyanatoethyl methacrylate.

**Patentansprüche**

1. Neue Zusammensetzung von Material, umfassend ein Urethandi(meth)acrylat, das erhältlich ist durch Umsetzen einer gesättigten Dihydroxyverbindung mit einem Diisocyanat und Bilden eines ersten Reaktionsprodukts mit ungefähr 2 Äquivalenten reaktivem Isocyanat, Umsetzen dieses ersten Reaktionsprodukts mit ungefähr 1 Äquivalent eines bevorzugt mit dem ersten Reaktionsprodukt reagierenden Hydroxyalkyl(meth)acrylats, um ein zweites Reaktionsprodukt zu bilden, Umsetzen des zweiten Reaktionsprodukts mit einem Glykol, um ein drittes Reaktionsprodukt zu bilden, und Umsetzen des

dritten Reaktionsprodukts mit einem Isocyanatoalkylen(meth)acrylat.

2. Neue Zusammensetzung von Material nach Anspruch 1, umfassend eine Verbindung der allgemeinen Formel

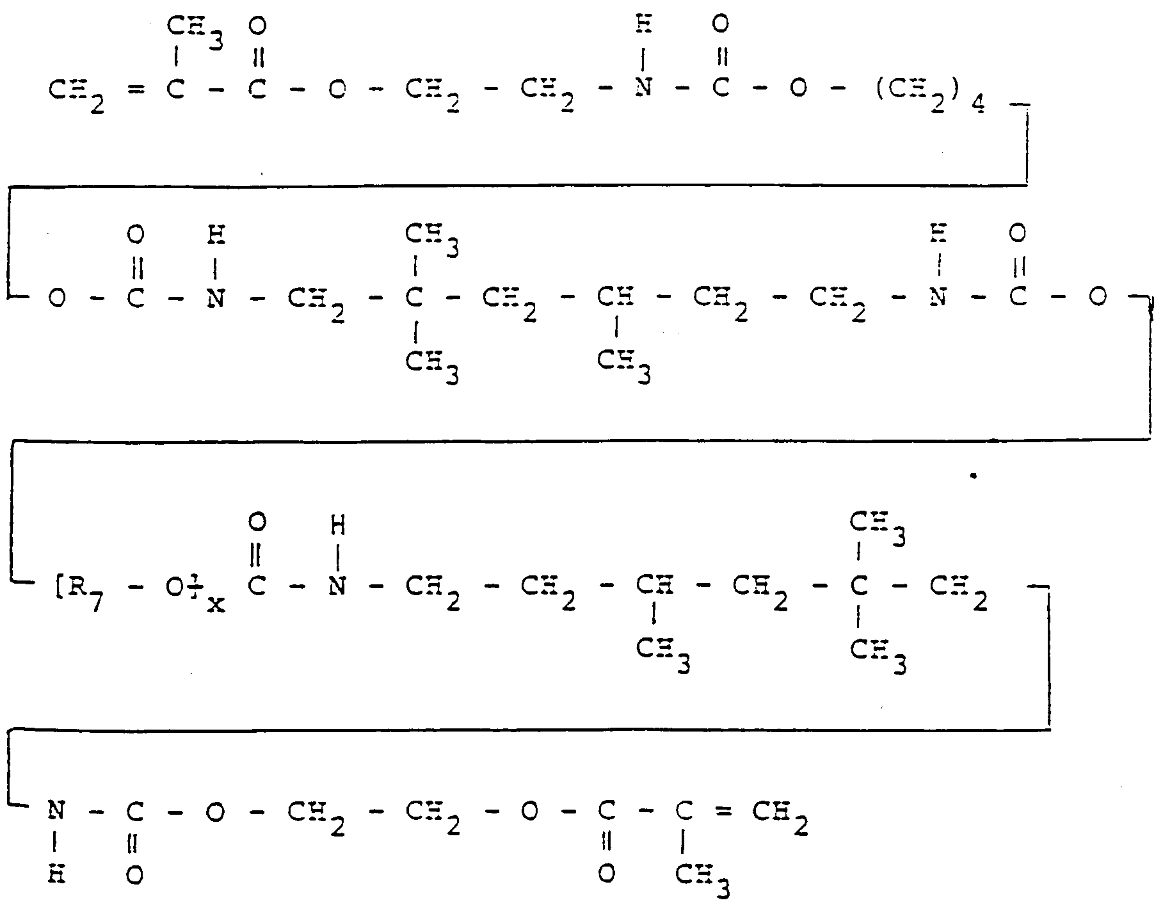

$R_7$ = Alkylen;
x = 10 bis 100.

3. Zusammensetzung nach irgendeinem der Ansprüche 1 oder 2, die als photohärtbares Zahnabdruckmaterial formuliert ist.

4. Neue Zusammensetzung von Material nach irgendeinem der Ansprüche 1 bis 3, umfassend einen Initiator, der durch aktinisches Licht im sichtbaren Bereich von 360 bis 600 nm aktiviert wird.

5. Verfahren zur Herstellung eines Urethandi(meth)acrylats, das zur Herstellung einer Zusammensetzung nach Anspruch 1 geeignet ist, umfassend das Umsetzen einer gesättigten Dihydroxyverbindung mit einem Diisocyanat und das Bilden eines ersten Reaktionsprodukts mit ungefähr 2 Äquivalenten reaktivem Isocyanat, das Umsetzen des ersten Reaktionsprodukts mit ungefähr 1 Äquivalent eines bevorzugt mit dem ersten Reaktionsprodukt reagierenden Hydroxyalkyl(meth)acrylats, um ein zweites Reaktionsprodukt zu bilden, das Umsetzen des zweiten Reaktionsprodukts mit einem Glykol, um ein drittes Reaktionsprodukt zu bilden, und das Umsetzen des dritten Reaktionsprodukts mit einem Isocyanatoalkylen(meth)acrylat.

6. Verfahren nach Anspruch 5, worin das Isocyanatoalkylen(meth)acrylat Isocyanatoethylmethacrylat ist.

**Revendications**

1. Nouvelle composition de matière comprenant un di(méth)acrylate d'uréthanne pouvant être obtenu par :

- réaction d'un composé dihydroxy saturé avec un diisocyanate et formation d'un premier produit de réaction ayant environ 2 équivalents d'isocyanate réactif,
- réaction dudit premier produit de réaction avec environ 1 équivalent d'un (méth)acrylate d'hydroxyalkyle réagissant de façon préférée avec ledit premier produit de réaction, afin de former un second produit de réaction,
- réaction dudit second produit de réaction avec un glycol, afin de former un troisième produit de réaction, et
- réaction dudit troisième produit de réaction avec un isocyanato alkylène (méth)acrylate.

**2.** Nouvelle composition de matière selon la revendication 1, comprenant un composé de la formule générale :

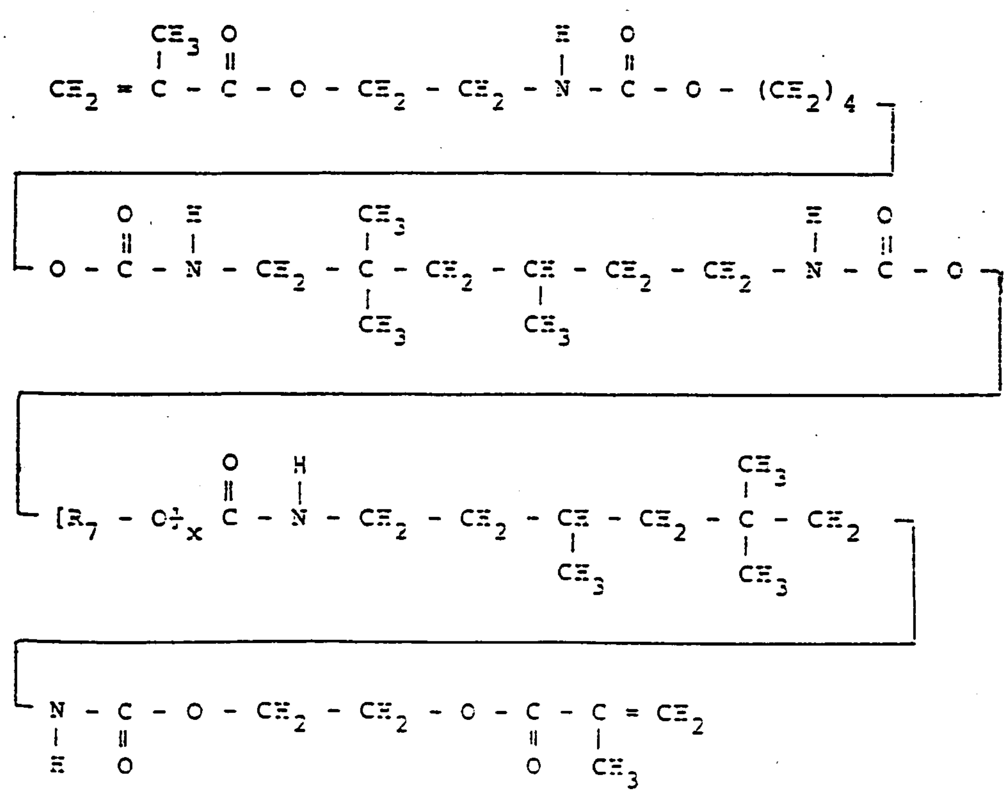

$R_7$ = alkylène ;
x = 10 à 100.

**3.** Composition selon l'une des revendications 1 et 2, qui est formulée en tant que matière photodurcissable pour empreintes dentaires.

**4.** Nouvelle composition de matière selon l'une des revendications 1 à 3, comprenant un initiateur activé par de la lumière actinique à l'intérieur de la plage de lumière visible de 360 à 600 nanomètres.

**5.** Procédé de fabrication d'un di(méth)acrylate d'uréthanne approprié à la fabrication d'une composition telle que définie à la revendication 1, comprenant

- la réaction d'un composé dihydroxy saturé avec un diisocyanate et la formation d'un premier produit de réaction ayant environ 2 équivalents d'isocyanate réactif,
- la réaction dudit premier produit de réaction avec environ 1 équivalent d'un (méth)acrylate d'hydroxyalkyle réagissant de façon préférée avec ledit premier produit de réaction, afin de former un second produit de réaction,
- la réaction dudit second produit de réaction avec un glycol, afin de former un troisième produit de réaction, et
- la réaction dudit troisième produit de réaction avec un isocyanato alkylène (méth)acrylate.

6. Procédé selon la revendication 5, dans lequel ledit isocyanato alkylène (méth)acrylate est le méthacrylate d'isocyanatoéthyle.